# EUROPEAN PATENT SPECIFICATION

(11) **EP 1 135 533 B1**
(45) Date of publication and mention of the grant of the patent: **21.04.2004**
(21) Application number: 99973075.7
(22) Date of filing: 01.12.1999
(51) Int. Cl.: C12Q 1/68

(54) **METHOD AND SYSTEM FOR DETECTING OLIGONUCLEOTIDES IN A SAMPLE**
VERFAHREN UND SYSTEM ZUR ERKENNUNG VON OLIGONUKLEOTIDEN IN EINER PROBE
PROCEDE ET SYSTEME PERMETTANT DE DETECTER DES OLIGONUCLEOTIDES DANS UN ECHANTILLON

(30) Priority: 01.12.1998 IL 12734698; 16.11.1999 IL 13296699
(43) Date of publication of application: 26.09.2001
(73) Proprietor: YISSUM RESEARCH DEVELOPMENT COMPANY OF THE HEBREW UNIVERSITY OF JERUSALEM, 91390 Jerusalem (IL)
(72) Inventor: WILLNER, Itamar, 90805 Jerusalem (IL); BAR DEA, Amos, 92623 Jerusalem (IL); PATOLSKY, Fernando, 99000 Beit Shemesh (IL); KATZ, Evgeny, 93262 Jerusalem (IL); DAGAN, Arie, 92341 Jerusalem (IL)
(74) Representative: Grünecker, Kinkeldey, Stockmair & Schwanhäusser Anwaltssozietät
(86) International application number: PCT/IL1999/000649
(87) International publication number: WO 2000/032813

(56) References cited:
- WO-A-92/08808
- US-A- 5 695 926
- OKAHATA Y. ET AL.,: "Kinetic measurement of DNA hybridization on an oligonucleotide-immobilized 27-MHz quartz crystal microbalance" ANAL. CHEM., vol. 70, - 1 April 1998 (1998-04-01) pages 1288-1296, XP000891733
- JENSEN K K ET AL: "KINETICS FOR HYBRIDIZATION OF PEPTIDE NUCLEIC ACIDS (PNA) WITH DNA AND RNA STUDIED WITH THE BIACORE TECHNIQUE" BIOCHEMISTRY,US,AMERICAN CHEMICAL SOCIETY. EASTON, PA, vol. 36, 1 January 1997 (1997-01-01), pages 5072-5077, XP002062488 ISSN: 0006-2960
- PATOLSKY F. ET AL.,: "Enzyme-linked amplified electrochemical sensing of oligonucleotide-DNA interactions by means of the precipitation of a insoluble product and using impedance spectroscopy" LANGMUIR, vol. 15, - 29 April 1999 (1999-04-29) pages 3703-3706, XP000901187

## Description

### FIELD OF THE INVENTION

The present invention relates to a method and system for the detection of oligonucleotides in a sample.

### PRIOR ART

The following is a list of prior art references which are relevant for a better understanding of the background of the invention:
1. Piunno, P.A.E., Krull, V.J., Hudson, R.H.E., Damha, M.J., Cohen, H., *Anal. Chim. Acta*, **288**:205-209, (1994).
2. Mandenius, C.F., Chollet, A., Lenburg, M.M., Lundström, I., *Anal. Lett.,* **22**:2961-2964, (1989).
3. Lidberg, B., Nylander, C., Lundström, I., *Sensors and Actuators*, **4**:299-302, (1993).
4. Jonsson, V., *Biotechniques*, **11**:620-624, (1991).
5. Mikkelsen, S.R., *Electroanalysis*, **8**:15-23, (1996).
6. Millan, K.M.; Sanauloo, A., Mikkelsen, S.R., *Anal. Chem*., **66**:3830-3833, (1994).
7. Hashimoto, K., Ito, K., Ishimori, Y., *Anal*. *Chem*., **66**:1236-1241, (1994).
8. Hashimoto, K., Ito, K., Ishimori, Y., *Anal*. *Chim. Acta,* **286**:219-224, (1994).
9. Wang, J., Palecek, E., Nielson, P.E., *J*. *Am. Chem. Soc*., **118**:7667-7670, (1996).
10. Ihara, T., Nakayama, M., Murata, K., Maeda, M., *Chem. Commun*., 1069-1070, (1997).
11. Bardea, A., Dagan, A., Ben-Dov, I., Amit, B., Willner, I., *Chem. Commun*., 839-840, (1998).
12. PCT Application No. WO 97/04314.

Acknowledgement of these references in the description below will be made by indicating the number from the above list.

### BACKGROUND OF THE INVENTION

The development of DNA-sensor devices attracts substantial recent research efforts directed to gene analysis, detection of genetic disorders, tissue matching and forensic applications. Optical detection of DNA was accomplished by the application of fluorescence labeled oligonucleotides^{(1,2)} or by the use of surface plasmon resonance^{(3,4)}. Electronic transduction of the formation of oligonucleotide complexes with a target DNA, and, particularly, in the quantitative assay of DNA is a major challenge of bioelectronics⁽⁵⁾. The organization of DNA-sensors requires the assembly of the sensing interface on a transducer, and the design of the appropriate electronic output that signals the formation of the recognition complex with the target DNA-analyte on the transducer element. Electrochemical DNA sensors based on the electrostatic attraction of electroactive transition metal complexes or organic dyes to oligonucleotide-DNA ds-complexes, e.g. Co(bpy)₃³⁺, acridin or Hoechst 33258 were reported⁽⁶⁻¹⁰⁾. Microgravimetric quartz-crystal- microbalance, QCM⁽¹¹⁾ analyses were also applied to sense the formation of complementary oligonucleotide-DNA complexes.

Two major difficulties are still encountered in the development of DNA sensors and relate to the sensitivity and specificity of the resulting sensing systems.

### GENERAL DESCRIPTION OF THE INVENTION

It is an object of the invention to provide a method and system for detecting target oligonucleotides in a sample.

The term "*detect*" or "*detection*" refers collectively to both a qualitative determination of the presence of the target oligonucleotide in the sample as well as at times for evaluation of the level of the target oligonucleotide in the sample.

In accordance with the first aspect of the invention there is provided a method for detecting a target oligonucleotide in a sample, comprising:
(a) providing a sensor device having a sensing interface carrying capturing oligonucleotides having each a nucleotide sequence complementary in at least a portion thereof to a first portion of the target oligonucleotide;
(b) providing verification oligonucleotides having each a nucleotide sequence complementary in at least a portion thereof to a second portion of the target oligonucleotide, other than said first portion;
(c) contacting the sample with the sensing interface under conditions which allow the target oligonucleotides, if present in the sample, to hybridize to the capturing oligonucleotides;
(d) prior to (c) or thereafter, allowing the verification oligonucleotides to hybridize to the target oligonucleotides if present in the sample; and
(e) detecting the presence of said verification oligonucleotides on the sensing interface.
   In accordance with another aspect, the present invention provides a system for detecting a target oligonucleotide in a sample, comprising:
(i) a sensor device having a sensing interface carrying capturing oligonucleotides having each a nucleotide sequence complementary in at least a portion thereof to a first portion of the target oligonucleotides;
(ii) verification oligonucleotides having each a nucleotide sequence complementary in at least a portion thereof to a second portion of the target oligonucleotide, other than said first portion; and
(iii) a combination comprising one or both of apparatus and reagents for detecting a verification oligonucleotide bound to the sensing interface.

The sample may be a biological specimen or a fractionation product thereof containing the oligonucleotides; a biological specimen treated to free and solubilized oligonucleotides; a specimen treated in a manner so as to digest nucleotide sequence into smaller oligonucleotides; a sample of oligonucleotides obtained by a PCR (Polymer Chain Reaction) process or any other oligonucleotide amplification process; etc.

In accordance with one embodiment, the present invention may be applied for a variety of genetic screening assays, such as, for example, screening intended to locate mutant genes.

In accordance with another embodiment, the invention may be applied for identifying pathogens in a sample.

There is a wide variety of assaying techniques available for detecting oligonucleotides which are based on hybridizing a probe oligonucleotide to the target oligonucleotide. Also known are assay techniques wherein a probe oligonucleotide is bound to a solid support which hybridize and "*fish out*" the target oligonucleotide from a tested sample. The invention is however unique in that it makes use of a verification oligonucleotide which increases both specificity and sensitivity of the assay.

In accordance with the invention, the verification oligonucleotide serves as an indicator for the presence of the target oligonucleotide in the sample. In other words, detection of an immobilized verification oligonucleotide on the surface is an indication that the target oligonucleotide is bound to the sensing surface and hence that it existed in the sample. In accordance with the invention there are thus two discrimination means to ensure specificity and sensitivity:
1. Hybridization of the target oligonucleotide to the capturing oligonucleotide on the sensing surface. The complementary sequence of the capturing oligonucleotide will typically, but not exclusively, comprise a number of oligonucleotides completing about one helix of the nucleotide strand, i.e. about twelve nucleotides. A complementary sequence of twelve oligonucleotides ensure on the one hand stable hybridization. On the other hand, a 12-mer oligonucleotide decreases the chance of binding to an incorrect oligonucleotide than a longer sequence. In the case the sample is a digested specimen of genomic DNA, or a fractionation product thereof comprising the oligonucleotides, there is some probability, which increases with the length of the capturing oligonucleotide, of binding to an incorrect oligonucleotide, namely an oligonucleotide other than the target oligonucleotide. This probability is lower, as aforesaid in the case of a shorter oligonucleotide. A sequence of about 12 nucleotides is preferred as it is optimal as far as ensuring binding stability, on the one hand, and reducing incorrect binding on the other hand. The invention is, however, not limited to such a capturing oligonucleotide.
2. Hybridization of the verification oligonucleotide to the target oligonucleotide.

These two independent binding events thus reduce the chance of false positive or false negative results.

The detection of the verification oligonucleotide on the sensing surface may be achieved by a number of means. In accordance with one embodiment of the invention, the sensor device comprises an electrochemical probe for electrical/ electrochemical measurements, e.g. for Faradaic impedance spectroscopy measurement or amperometric detection of the oligonucleotide. In addition, detection may also be carried out by a number of other electrochemical techniques known *per se* based on the control of interfacial electron transfer rates between the sensing interface and the surrounding medium. For this electrochemical embodiment of the invention, the sensing surface is formed on a conductive matrix on which the capturing oligonucleotides are bound. Such an electrically conducting matrix may for example be made or coated by a metal such as gold, platinum, silver or copper.

In accordance with another embodiment of the invention, the sensing device is a quartz crystal microbalance (QCM) probe in which case the presence of the verification oligonucleotide on the sensing surface is based on measurement of changes in resonance frequency of the probe. Microgravimetric QCM techniques are known *per se*, and are described, for example, in PCT Application WO 97/04314⁽¹²⁾.

In accordance with one preferred embodiment of the invention, the verification oligonucleotide is conjugated to a recognition agent which specifically binds to a signal-amplifying agent. The signal-amplifying agent, according to this embodiment, comprises a recognition partner capable of specific binding to the recognition agent. The recognition agent and the recognition partner constitute together a recognition couple. In accordance with another preferred embodiment of the invention the verification oligonucleotide is bound to or complexed directly with a signal-amplifying agent.

The recognition couple may, for example, be one of the couples selected from the group of biotin-avidin or biotin-streptavidin, receptor-ligand, sugar-lectin, antibody-antigen (the term "*antibody*" should be understood as referring to a polyclonal or a polyclonal antibody, to a fraction of an antibody comprising the variable, antigen-biotin binding portion, etc.). The recognition agent may be one member of the aforementioned couples, while the recognition partner may then be the other member of the recognition couple.

In accordance with one embodiment of the invention, the verification oligonucleotide comprises a first recognition agent and the signal-amplifying agent comprises a second recognition agent, with the first and the second recognition agents being the same or different, and both being capable of specific binding to a recognition partner to form a recognition couple. The recognition partner is thus capable of specific binding to both the first and the second recognition agents and thus its introduction to a sensing surface to which the verification oligonucleotide has bound, will yield binding of the signal-amplifying agent to the sensing interface. An example of a recognition partner is avidin or streptavidin, with both the first and second recognition agents being biotin. In accordance with an embodiment of the invention, the signal-amplifying agent comprises a plurality of said second recognition agents and thereby, by a sequence of exposures of the sensing interface to said recognition partner and said signal-amplifying agent, a complex comprising two or more signal amplifying agents bound to each verification oligonucleotide on the sensing interface, may thereby be obtained to yield an increased signal amplification.

The signal-amplifying agent, according to one embodiment of the invention is a moiety or particle which directly increases the mass immobilized on the sensing surface. The signal-amplifying agent may, for example, comprise molecules, a super molecular structure, or particle, e.g. colloid particles, macromolecules, clusters or molecules, liposomes, etc. In addition, the signal-amplifying agent may also be conjugated to or complexed with a label including, but not limited to an enzyme label. In case of an enzyme label, the enzyme is of a kind that can catalyze a reaction giving rise to an insoluble product. In accordance with this embodiment, the enzyme, after the signal-amplifying agent binds to the recognition agent, is allowed to catalyze a reaction which gives rise to the insoluble product, and the product then precipitates onto the sensing surface. This product may then be detected by a variety of electric-electronic or optical detection means. In an assay carried out in accordance with the electrochemical embodiment, such a precipitate is preferably detected by the large change in electrode impedance resulting therefrom or, alternatively, it may be detected by the mass change on a piezaelectric crystal resulting in a frequency change of the crystal.

In accordance with an embodiment of the invention, a particle, serving as a single-amplifying agent by its own right, may also carry an enzyme for further amplification of the binding-associated signal. For example, a liposome used as a signal-amplification agent may be bound to or complexed with said enzyme to allow further increase in mass as a result of precipitation of the enzyme-catalyzed insoluble product on the sensing surface, and thus a further amplified binding- related signal.

The invention also provides, for use in the above method and system, one or more reagents, selected from the group consisting of:
(i) said verification oligonucleotide;
(ii) an amplifying agent for amplifying the signal resulting from binding of said verification oligonucleotide to said sensing interface.

The invention will now be described with reference to a non-limiting specific embodiment. As will no doubt be appreciated, this description is a mere illustrative example of the wider scope of the invention as defined in the appended claims.

### BRIEF DESCRIPTION OF THE DRAWINGS

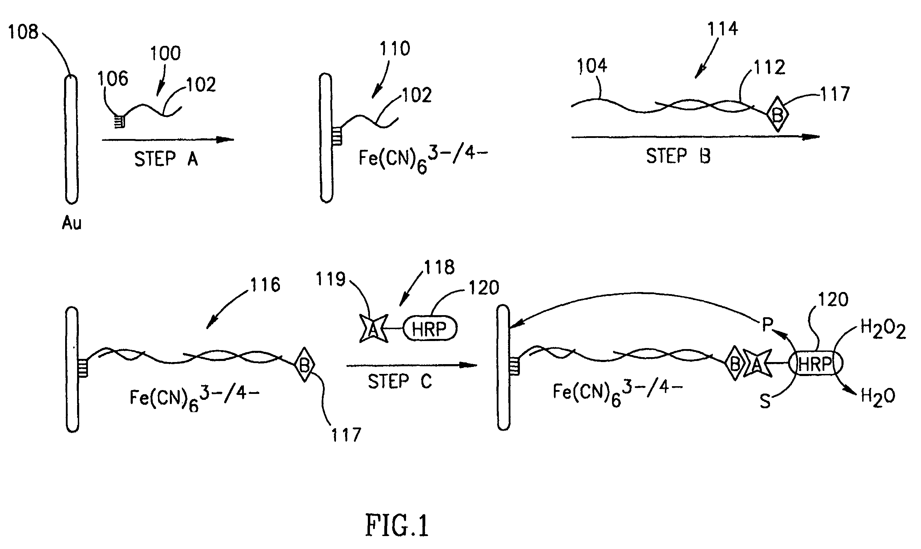

**Fig. 1** illustrates a bioelectronic system in accordance with an embodiment of the invention including assembly of the sensor and its use in detection of an target oligonucleotide in a sample.

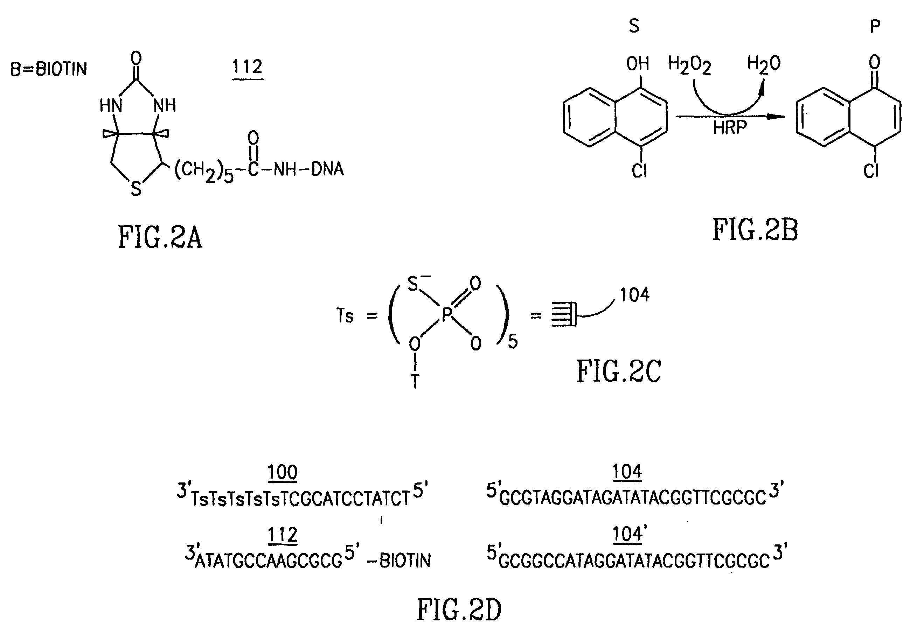

**Figs. 2A-2D** illustrate in somewhat more details some of the components of the system of Fig. 1 as used in the exemplary experiments: Fig. 2A shows a DNA strand covalently bound to biotin; Fig. 2B illustrates the chemical reaction, catalyzed by horseradish peroxidase (HRP), in which 4-chloro- naphthol is reacted to form an insoluble product; Fig. 2C shows the structure of the thiophosphate thymine; and Fig. 2D shows the sequence of some oligonucleotides used in the exemplary experiments.

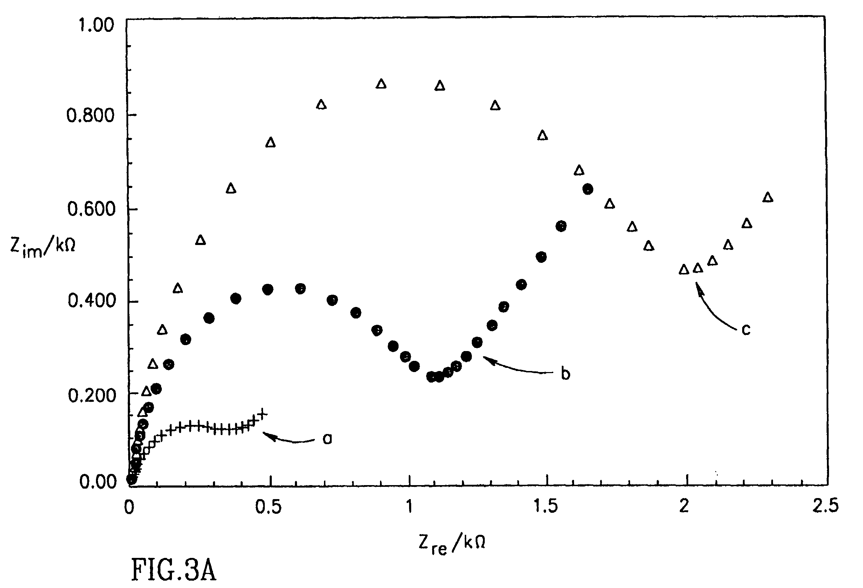

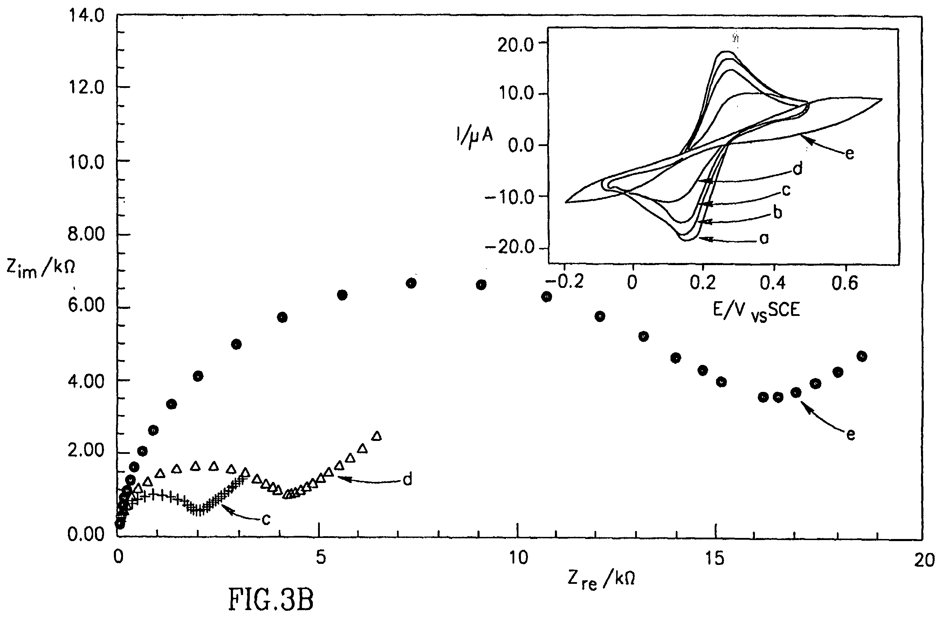

**Figs. 3A** and **3B** show the impedance features, presented as Nyquist plots, of a bare electrode (curve a), after functionalization of the electrode with the capturing oligonucleotide (curve b), after binding the target DNA and the biotinylated oligonucleotide hybrid (curve c), after interaction with the avidin-HRP conjugate (curve d) and after some period of catalysis of the enzyme resulting in deposit of insoluble product on the sensing surface (curve e). It should be noted that Figs. 3A and 3B are of the same experiment but drawn to different scales.

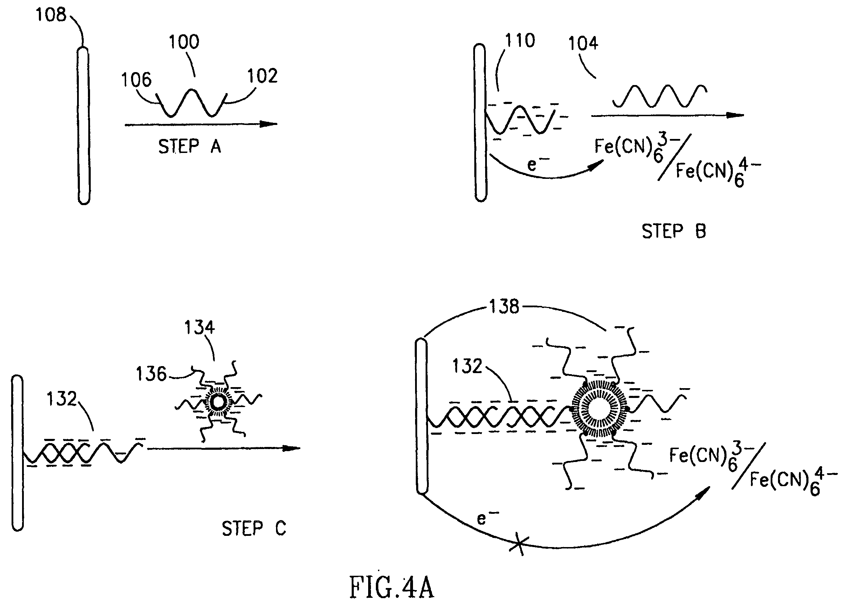

**Fig. 4A** illustrates a bioelectronic system in accordance with a further embodiment of the invention including assembly of the sensor and its use in detection of a target oligonucleotide in a sample.

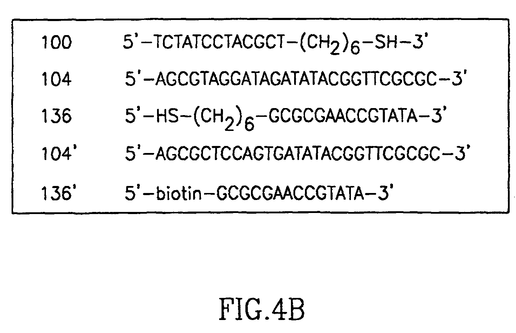

**Fig. 4B** shows the sequence of the different oligonucleotides used in a system of the kind illustrated in Fig. 4B, in the accompanying experiments.

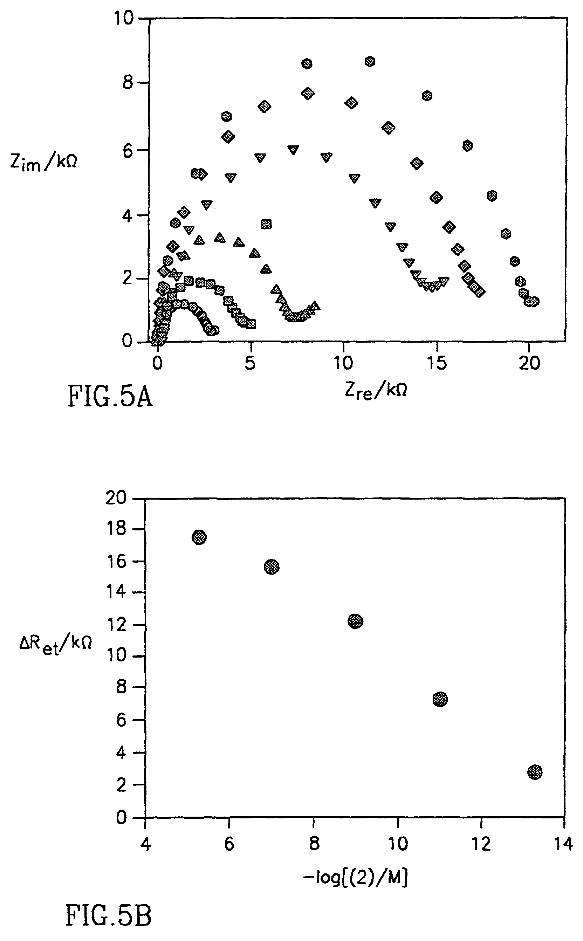

**Figs. 5A** shows the impedance spectra presented as Nyquist plots of the feature illustrated in Fig. 4, the spectra including: a functionalized electrode carrying the capturing oligonucleotides (curve a); after interacting the functionalized electrode with a sample carrying the target oligonucleotide (curve b); after interaction with the oligonucleotide-functionalized liposome (curve c); after interacting an electrode functionalized with a mutated capturing oligonucleotide (curve d); and after treatment of the mutated capturing oligonucleotide bearing electrode with funcionalized liposome (curve e).
**Fig. 5B** shows the changes in electron transfer resistance of a functionalized electrode upon treatment with different concentrations of the target oligonucleotide and amplification with labeled liposomes.

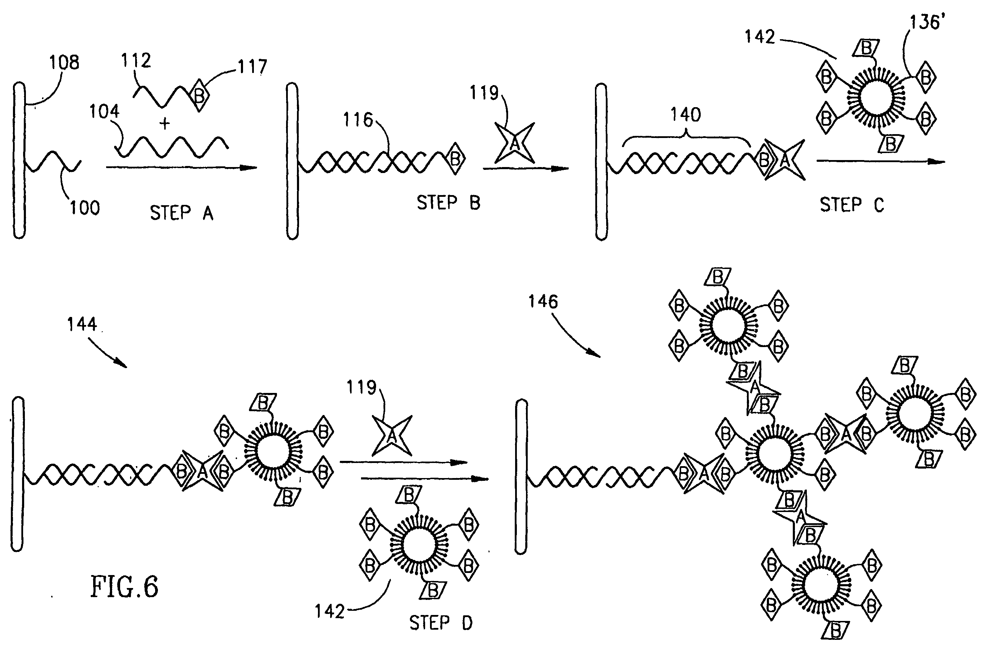

**Fig. 6** illustrates a bioelectronic system in accordance with a yet further embodiment of the invention including assembly of the sensor and its use in detection of a target oligonucleotide in a sample.

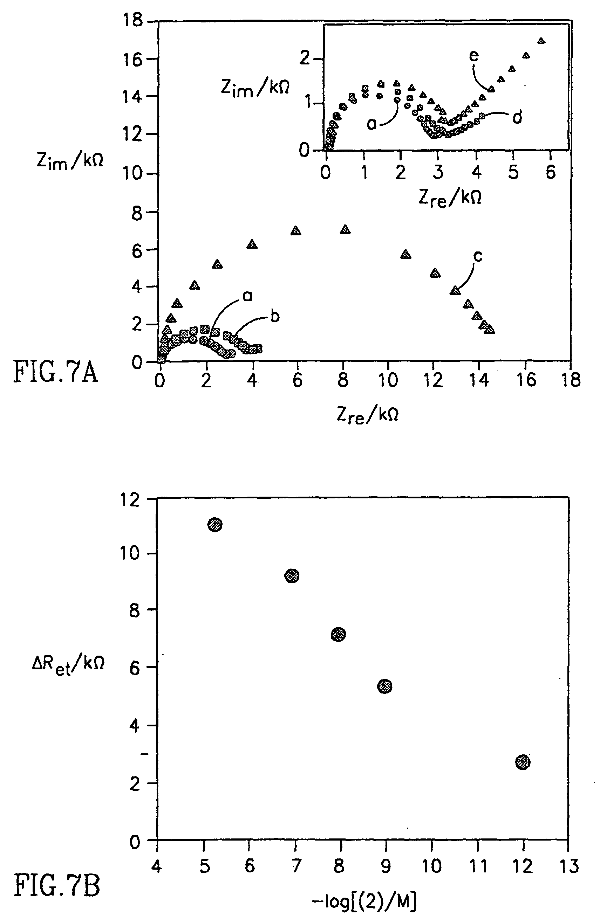

**Fig. 7A** shows the impedance spectra presented as Nyquist plots of the features illustrated in Fig. 6, the spectra including: a functionalized electrode (curve a); after interaction with a sample carrying the target oligonucleotide pre-treated with a biotin-labeled verification oligonucleotide (curve b); after subsequent interaction with avidin (curve c); after subsequent interaction with biotinylated liposomes (curve d); after subsequent interaction for a second time with avidin (curve e); and after a subsequent interaction for a second time with the biotinylated liposomes (curve f).
**Fig. 7B** shows a calibration curve which corresponds to the changes in the electron transfer resistances of the functionalized electrode upon interaction with different concentrations of the target oligonucleotide and enhancement of the detecting processes by a double-step avidin/biotinylated liposome amplification path.

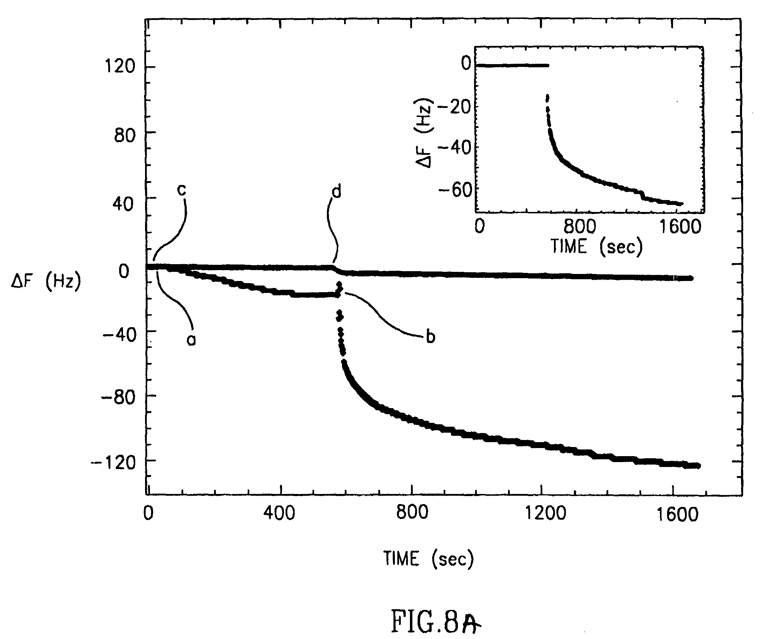

**Fig. 8A** shows time-dependent frequency changes of oligonucleotide-bound crystal after interaction with a sample containing the target oligonucleotide (curve a); after interaction of the resulting electrode with an oligonucleotide-labeled liposome (curve b); after functionalizing a crystal with a mutated capturing oligonucleotide (curve c); after bringing into contact the mutated oligonucleotide-labeled crystal with oligonucleotide-labeled liposome (curve d); after treating an oligonucleotide-labeled crystal with oligonucleotide-labeled liposomes (curve e).

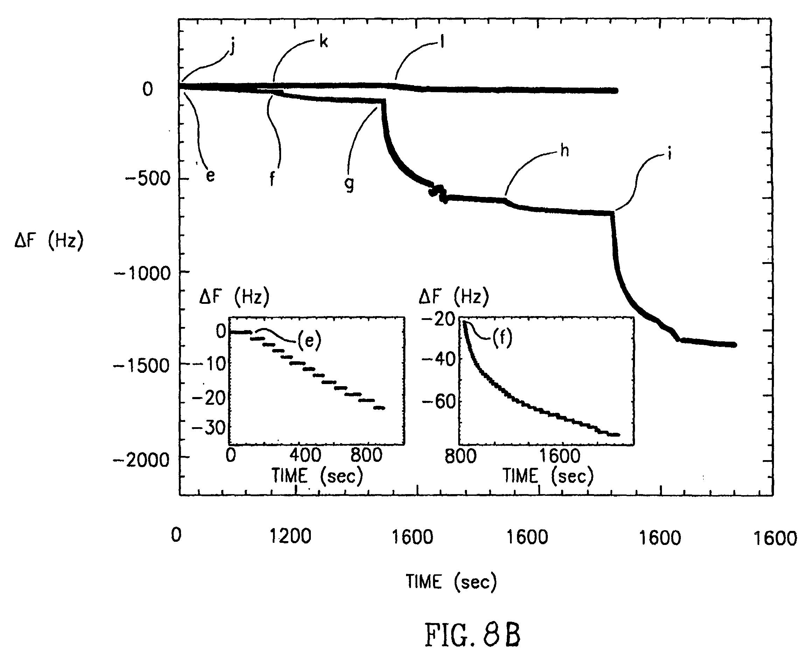

**Fig. 8B** shows time-dependent frequency changes of oligonucleotide-labeled crystal after interacting with a sample containing the target oligonucleotide (Curve e); after interacting the resulting interface with avidin (curve f); after interacting the resulting assembly with biotinylated liposome (curve h); after further interacting with avidin (curve g); after further interacting with biotinylated liposome (curve i); after treating an oligonucleotide labeled crystal with a mutated oligonucleotide pre-treated with biotinylated liposomes (curve j); after treating the resulting interface (of curve j) with avidin (curve k); and after treating with biotinylated liposomes.

### DETAILED DESCRIPTION OF A PREFERRED EMBODIMENT

The manner of assembly of the DNA sensor in accordance with an embodiment of the invention and its use, are illustrated in Fig. 1. Oligonucleotide **100**, serving as the capturing oligonucleotide, includes a first portion **102**, typically a 12-base sequence, that is complementary to a first portion of the target oligonucleotide, and a second portion **106** for binding to the electrode, e.g. a gold (Au) electrode **108**. The binding portion **106** may, for example, be a several base (e.g 5) thiosphosphate thymidine (TS) sequence, illustrated in Fig. 2C. Occasionally the two portions **102**, **106** may be separated by one or more separator base-nucleotides. The electrode **108** and oligonucleotide **100** are reacted such that portion **106** binds to the surface of the Au electrode. As a result, functionalized electrode with a sensing surface **110** is formed.

A verification oligonucleotide **112** is contacted with a sample which contains the target oligonucleotide **104** whereby a partial double-stranded structure **114** is formed. This structure is then contacted with the sensing surface (step B) yielding a bifunctional double-stranded oligonucleotide assembly **116**. It should be noted that it is possible in accordance with another embodiment of the invention to first contact the sensing surface **110** with the sample and only then bring a reagent solution which comprises the verification oligonucleotide **112** into contact with the sensing surface. This will first yield binding of the target oligonucleotide **104** (if present in the sample) to the sensing surface and then binding of verification oligonucleotide **112** to yield assembly **116**. In both cases, the presence of the verification oligonucleotide **116** on the sensing surface serves as an indication of the presence of the target oligonucleotide **104** in the sample.

The detection of the verification oligonucleotide on the sensing surface may be achieved by a number of means some of which were explained above. For example, the verification oligonucleotide may carry a label which may be detected electrically, e.g. by determining change in impedance, or electron transport between the electrode **108** and the surrounding medium. The label, by one embodiment, is an enzyme which can catalyze a reaction yielding an insoluble reaction product which precipitates on the surface's electrode thus increasing impedance. This is illustrated in step C of Fig. 1.

In accordance with one embodiment, verification oligonucleotide **112** is bound to a biotin moiety **117**. A label complex **118** which comprises an avidin **119** bound to an enzyme **120** is contacted with the sensing surface (step C) resulting in binding of complex **118** to the sensing surface. Enzyme **120** can catalyze a reaction converting a substrate (**S**) into an insoluble product (**P**) which is thus deposited on the sensing surface. Both the binding of the labeling complex **118** to the sensing surface as well as the precipitation of product (**P**) onto the sensing surface can be monitored similarly as above (i.e. change of impedance or a change of mass in the case of QCM-type measurement).

In Figs. 4A, 4B and 6, the same reference numerals as used in Fig. 1 are used for like components.

According to another embodiment illustrated in Fig. 4A, use is made with a verification oligonucleotide-modified liposome **134** that is bound to the double-stranded immobilized assembly **132**, to form an immobilized double-stranded oligonucleotide-liposome assembly **138**. The binding of the labeled verification liposome onto the sensing surface can be monitored as described above.

The sensing of the target oligonucleotide **104** in accordance with a further embodiment, can be further amplified by using a double-step avidin/biotin-labeled-liposome amplification pathway as shown schematically in Fig. 6. Functionalized electrode **110** is first hybridized with target DNA **104** pre-treated with biotin-labeled oligonucleotide **112** having a portion sequence complementary with oligonucleotide **100**, immobilized on said electrode **108**, to form bifunctional double-stranded biotinylated assembly **116**. The formed assembly is then reacted with biotinylated liposomes **142** to form a liposome containing assembly **144**. This assembly can further be reacted with avidin and additional biotinylated liposomes, to yield a multi-liposome assembly **146**.

The invention will now be further illustrated by the following example:

### EXAMPLES

For clarity, in the description below the same reference numeral to those used above will be used. However, by doing so, it should not, in any way, limit the scope of the invention to the specific examples below.

It should be noted that the scheme shown in Fig. 1 can be employed for various different assays than that specifically exemplified herein. Furthermore, a similar scheme, *mutatis mutandis*, may also be used for assaying a target oligonucleotide in other assay techniques, e.g. microgravimetric QCM. In this latter case rather than electric/electronic measurements, the measurement is of change in resonance frequency of the piezoelectric crystal as a result of mass change.

### Example 1 Enzyme-amplified detection of a target oligonucleotide in a sample

The sensor preparation sequence as used in the Example can be seen in Fig. 1, while the sequences of the oligonucleotides used can be seen in Fig. 2D. In Fig. 2D each oligonucleotide is identified by the reference numerals used in the example.

An 18-mer oligonucleotide **100** (SEQ ID NO: 1) which included a 12-base sequence **102** that is complementary to a part of the analyte, the Tay-Sachs (TS) mutant **104** (SEQ ID NO: 2) was used. In addition, oligonucleotide **100** included a 5-base thiophosphate thymine-TS tag **106** for its assembly on the gold (Au) electrode **108**, and a single T-base separating the tag from the sensing oligonucleotide sequence. A disc Au-electrode **108**, 0.05 cm², was interacted with oligonucleotide **100** (20 µM, 10 hours) resulting in the assembly of the sensing interface on the gold support (step A in Fig. 1). The resulting functionalized electrode **110** was interacted with a solution that included the target analyte, the TS-mutant sequence **104** (5.8 x 10⁻⁷ g/mL⁻¹, 4 hours), and a biotinylated verification oligonucleotide **112** (SEQ ID NO: 3, bound via the 5' end to biotin, Fig. 2D), 2 x 10⁻⁵ g/mL⁻¹ (step B in Fig. 1).

Verification oligonucleotide **112** is complementary to one portion of an oligonucleotide **104** and consequently these two oligonucleotides hybridize to form a partial double-stranded structure **114**. Target oligonucleotide **104** has another sequence complementary to portion **102** of capturing oligonucleotide **100** and thus step B results in the formation of a bifunctional double-stranded DNA-oligonucleotide assembly **116**.

Sensing surface with bifunctional double-stranded DNA- oligonucleotide assembly **116** is then treated with an avidin labeled with horseradish peroxidase (HRP) (1 x 10⁻⁸ g/mL⁻¹, 3 hours) (step C in Fig. 1). HRP can catalyze the oxidation of 4-chloro-1-naphthol (**S**) by hydrogen peroxide (H₂O₂) giving rise to the formation of an insoluble product (**P**) which precipitates on the electrode. Other enzyme-substrate couples yielding an insoluble product which may be used include: alkaline phosphatase and indoyl phosphate derivatives as substrates; glucose oxidase and tetrazolium salts as substrates; etc.

As the oligonucleotide and oligonucleotide-DNA layered assemblies are negatively charged, the electrostatic repulsion of a negatively-charged redox-probe, e.g. Fe(CN)₆^{3-/4-}, from the electrode support is anticipated to perturb the interfacial electron transfer. This is expected to introduce an electron transfer resistance that can be detected by Faradaic impedance spectroscopy or other electrochemical means such as reduction of the amperometric response of the electrode. The biocatalytic precipitation of the product (**P**) on the electrode is expected to further insulate the conductive support and to lead to a high interfacial electron transfer resistance or a reduction of the amperometric response of an electroactive species solubilized in the medium surrounding the electrode.

Fig. 3A shows the impedance features, using Fe(CN)₆^{3-/4-} as redox- probe, presented as Nyquist plots (Zᵢₘ vs. Zᵣₑ), of the bare electrode **108** (curve a), of the functionalized electrode with the sensing surface **110** (curve b) and of the layered bifunctional double-stranded oligonucleotide-target DNA and biotinylated oligonucleotide assembly (curve c). The respective semicircle diameters correspond to the interfacial electron transfer resistnaces, Rₑₜ. It can be seen that the electron transfer resistance increases upon the build-up of the biotinylated oligonucleotide-DNA assembly. For example, for the functionalized electrode Rₑₜ = 1.1 kΩ whereas Rₑₜ is increased to about 2.2 kΩ upon the association of the complex **114**. These results are consistent with the fact that the negative charge increases upon the two-step organization of the assembly. This results in the enhanced electrostatic repulsion of the redox-probe, and introduces higher interfacial electron transfer resistance.

Fig. 3B shows the impedance spectra of the bifunctional double-stranded assembly consisting of the target DNA linked to the sensing interface and the biotinylated oligonucleotide, before (curve c) and after (curve d) interaction with the avidin-HRP conjugate. Upon the association of the avidin-HRP biocatalytic conjugate to the layer, a considerable increase in the electron transfer resistance is observed due to the partial insulation of the electrode by the proteins. In the presence of H₂O₂ and the substrate (**S**), the biocatalytic precipitation of the product onto the electrode occurs. This insulates the conductive support, resulting in a very high increase in the electron transfer resistance, curve (e), Rₑₜ = 17 kΩ. It should be noted that the two parameters controlling the sensitivity of the DNA-sensing devices are the time of incubation of the functionalized-monolayer-electrode **110** with the complex **114** and more important, the time-interval used to precipitate the product by the avidin-HRP biocatalyic conjugate. Using this configuration, and upon precipitation of **P** for 40 min. it was possible to sense the target DNA **104** at a concentration of 20 x 10⁻⁹ g/mL⁻¹, Rₑₜ = 7.9 kΩ.

Control experiments show that the oligonucleotide sensing assembly has a high specificity and selectivity. Treatment of the functinalized electrode **110** with the biotinylated oligonucleotide **112** and then with the avidin-HRP conjugate **118**, but without the interaction with the target DNA **104**, yielded only a minute change in the electron transfer resistance.

In order to test the specificity of the system, the same assay was performed with a DNA fragment **104'** (SEQ ID NO: 4) that corresponds to the normal gene sequence in which the 7-based mutation leads to the TS-genetic disorder. After contact of the sensing interface with a complex between fragment **104'** and the verification oligonucleotide **112**, the system was subjected to the biocatalytic precipitation process using the avidin-HRP conjugate, using the same protocol as illustrated in Fig. 1. However, no noticeable changes in the electron's transfer resistance at the electrodes were observed, implying that the lack of formation of a complex between the capturing oligonucleotide on the sensing surface and the complex formed between the target oligonucleotide **104** and the verification oligonucleotide **112** which prevented the subsequent formation of the precipitant layer on the electrode.

Cyclic voltammetry experiments (see insert Fig. 3B) further confirm the stepwise organization of the bifunctional double-stranded complex **116**, and that the precipitation of the insulating layer formed by product **P** on the electrode, gradually perturb the electron-transfer kinetics of Fe(CN)₆³⁻. Fig. 3B inset, shows the cyclic voltammograms of Fe(CN)₆³⁻ at a bare Au- electrode (curve a), upon formation of the sensing assembly **110** (curve b), and upon the formation of the double-stranded assembly **110** (curve c). The stepwise assembly of the layers is accompanied by a decrease in the amperometric response of the electrode and an increase in the peak-to-peak separation between the cathodic and anodic waves of the redox-probe. This is consistent with the enhanced electron transfer barriers introduced upon the assembly of the negatively-charged oligonucleotide assembly. Association of the avidin-HRP conjugate onto the layer (curve d), further separates the redox waves of Fe(CN)₆³⁻ implying that binding of the protein insulates the electrode and perturbs the interfacial electron transfer. Biocatalytic precipitation of **P** onto the electrode insulates the conductive support, and the electrical response of the redox-probe is almost entirely blocked, (curve e). The result shown in the inset of Fig. 3B demonstrates that amperometric transduction of the formation of the complex **116,** binding of avidin-linked HRP **118,** and further precipitation of the product **P** is possible.

By some modification of the assayed scheme described above, rather than determining the formation of the insoluble product precipitates on the electrode by means of a Faradaic impedance spectroscopy, it may also be determined by means of amperometric detection, by optical means and others or by microgravimetric QCM detection, an example for the latter being provided hereinbelow.

### Example 2 Liposome-amplified detection of an oligonucleotide

The sensor preparation sequence as used in the example can be seen in Fig. 4A, while the sequence of the oligonucleotides used can be seen in Fig. 4B. In Fig. 4B, each oligonucleotide is identified by the reference numeral as used in the examples.

A mercaptohexyl oligonucleotide **100** (SEQ ID NO: 5 bound to the mercaptohexyl via the 3' end) including a portion **102** that is complementary to a part of the analyte **104** (SEQ ID NO: 6) and the mercapto-derived portion **106** for its assembly as a monolayer on an Au-electrode **108** was used as a capturing agent. The mercaptohexyl oligonucleotide **100** was assembled on the Au-electrode **108** as a monolayer, to obtain the sensing interface **110** (step A in Fig. 4A). A surface coverage of the electrode of 1.1x10⁻¹¹ mole/cm² was determined by Tarlov's electrochemical method [Tarlov M.J. *et al*. Anal. Chem. **70**:4670 (1998)], and comparable results were obtained by QCM analyses. The resulting monolayer-functionalized electrode **110** was then brought into contact with a sample containing the target analyte, oligonucleotide **104** (5x10⁻⁶ M, 15 hours incubation, 25°C), to yield a double-stranded assembly **132** (step B in Fig. 4A) wherein at least part (**130**) of the assembled analyte is left free for further hybridization. The resulting electrode interface was then treated with oligonucleotide-labeled liposome **134** (lipid concentration 0.2mM, 15 min. 25°C). The oligonucleotide moiety **136** (SEQ ID NO: 7, bound to a mercaptohexyl group via the 3' end, Fig. 4B) within the labeled liposome **134** is complementary to the residual base-sequence **130** of the analyte. Thus, a liposome-linked three-component double-stranded assembly **138**, consisting of the capturing agent **100**, the analyte **104**, and the liposome tagged with oligonucleotide **136**, is generated on the electrode support.

The oligonucleotide-labeled-liposome was prepared by the assembly of liposomes that are composed of phosphatidic acid, phosphatidyl choline, maleimide-phosphatidylethanolamine, cholesterol (marked with 3H-cholesterol, 45 Ci/mole) at a ratio of 79:20:1:0.1, that were modified with oligonucleotide **136** by incubation therewith for 20 hours at 4°C and purified by chromatography (Sephadex G-75). The surface coverage of the liposome with oligonucleotide **136** (50-60 oligonucleotide units per liposome) was determined by reacting the resulting liposomes with Oligreen (Molecular probe) and following fluorescence intensity of the resulting liposome suspension at λ=480nm. The size of the liposomes was determined by dynamics light-scattering and corresponded to 220±20 nm.

The oligonucleotide-labeled liposomes **134** are negatively charged in order to eliminate non-specific adsorption of the liposomes onto the sensing interface. The liposomes associated with the electrode support represent "giant" negatively charged amplifying agents that electrostatically repel a negatively charged redox-probe stabilized in the electrolyte solution. That is, the biorecognition event between the capturing oligonucleotide **100** and the target oligonucleotide **104** is amplified by the generation of a highly-charged microenvironment that repels the electroactive probe, Fe(CN)₆^{3-/4-}, in solution. The electron transfer resistance produced by the assembly **138** was then assayed by Faradaic impedance spectroscopy.

Fig. 5A shows the impedance spectra (in the form of Nyquist plots, Zᵢₘ vs. Zᵣₑ) of oligonucleotide-functionalized electrode **110** (curve a) after hybridization with the target oligonucleotide **100** to form the layered double-stranded oligonucleotide assembly **132** (curve b), and after interaction with the probing oligonucleotide-labled liposome **136** to form the amplified assembly **138** (curve c). While a bare Au-electrode exhibits an electron transfer resistance of 0.5 kΩ, the associated of the capturing oligonucleotide **100** onto the conducting support increased the electron transfer resistance to 3 kΩ. This is attributed to the electrostatic repulsion of the redox label, Fe(CN)₆^{3-/4-}, that results in a barrier for the interfacial electron transfer. The formation of the double-stranded assembly with the target oligonucleotide increased the electron-transfer resistance to Rₑₜ=4.5 k Ω. This is consistent with the results presented by Example 1, hereinabove and with the fact that the higher negative charge formed on the surface as a result of hybridization, enhances the electrostatic repulsion of the electroactive species on the solution. Binding of the oligonucleotide-modified liposome **134** introduced a very high electron transfer resistance corresponding to 15 kΩ. This result is attributed to the formation of a negatively charged micro-interface upon the association of the liposome to the double-stranded assembly.

A control experiment, for the evaluation of the system's specificity, was conducted, which included an attempt to detect the presence of an oligonucleotide **104'** (SEQ ID NO: 8), that included a 6-base mutation relative to the target DNA **104**. Fig. 5A further shows the impedance spectrum of the functionalized-electrode **110** after its treatment with the mutant **104'** (curve e) and the impedance spectrum of the resulting electrode after further treatment with the oligonucleotide-labeled liposome **134** (curve e). As may be understood from the results presented in Fig. 5A, the interfacial electron transfer resistances were almost unchanged in this control experiment, implying that the sensing interface is selective for analyses of target oligonucleotide **104**. The results also indicate that no non-specific association of mutant **104'** or of the oligonucleotide-labeled liposomes **134** on the electrode took place. This is attributed to the electrostatic repulsion existing between these components and the sensing interface.

The extent of increase in the electron transfer resistance upon the binding of the analyte-oligonucleotide, and the secondary association of the modified liposome is controlled by the bulk concentration of the analyte, as shown in Fig. 5B. the lower sensitivity limit for analyzing the analyte DNA was determined to be 1.2x10⁻¹² M at a signal-to-noise value of S/N = 3.

A further control experiment, where only the oligonucleotide **136** interacted with the double-stranded assembly of the capturing oligonucleotide **100** and the target oligonucleotide **104** introduced only a small increase in the electron transfer resistance, Rₑₜ = 4.7 kΩ., indicating that the negatively charged liposome indeed amplified the electrostatic repulsion of the redox label.

The sensing system may be further amplified as schematically illustrated in Fig. 6, wherein the presence of target oligonucleotide **104** (SEQ ID NO:1) was detected using the negatively-charged liposomes **142** carrying the biotinylated oligonucleotide **136'** (SEQ ID NO:9 bound via the 5' end to biotin). Accordingly, oligonucleotide-functianalized electrode **110** is reacted with the target oligonucleotide (5x10⁻⁶ M, 15 min. of hybridization, at 25°C), pre-treated with biotinylated verification oligonucleotide **112** (SEQ ID NO:5) 1x10⁻⁵ M, interaction time 2 hr. 25°C), being complementary to segment **102** of the target oligonucleotide (step A, in Fig. 6). This process results in a three-component double-stranded-assembly on the electrode, consisting of the capturing oligonucleotide **100**, the analyte oligonucleotide **104** and the biotin-labeled oligonucleotide **112**. Association of avidin **118** (8 min. of incubation, step B in Fig. 6) and then the biotin-tagged-liposome **142** (8 min. of incubation, step C in Fig. 6) resulted in the formation of a negatively-changed interface **144** that amplified the primary oligonucleotide recognition event by the electrostatic repulsion of Fe(CN)₆^{3-/4-} and the enhancement of the interfacial electron transfer resistance. This sensing configuration enabled the further amplification of the biorecognition event by the multiple reaction of the resulting array with avidin and then with the biotinylated liposomes to yield a dense array of the negatively-charged liposomes. The biotin-labeled liposomes were composed of phosphatidyl choline, phosphatidylethanolamine, cholesterol (marked with 3H-cholesterol, 45Ci/mole) and biotinylated phosphatidylethanolamine with a ratio corresponding to 80:20:0.1:0.1. The average coverage of the liposomes with biotin corresponded to 550, which were purified by gel chromatography (DEAE Sephadex A-25). The size of the liposomes was determined by dynamic light scattering to be 180±40 nm.

Fig. 7A shows the impedance spectra of the array in the different steps of modification. The oligonucleotide-functionalized interface **108** exhibited an electron transfer resistance corresponding to 3 kΩ (curve a), and upon the formation of the double-stranded assembly with the analyte-DNA **104** complexed with the biotinylated oligonucleotide **112** to form immobilized biotinylated analyte **116,** the electron transfer resistance increased to Rₑₜ = 4.8 kΩ. (curve b). Association of avidin **119** (2.5µg/ml) to the interface **116** further increased the electron transfer resistance to 7.6 kΩ, as a result of the hydrophobic, insulating features of the protein (curve c). Association of the biotin-labeled liposome to the sensing surface 30 min. lipid concentration 0.25 mM), substantially increased the electron transfer resistance, Rₑₜ = 14.8 kΩ. (curve d).

The sensing of the target-DNA was further amplified by the application of a second step of association of the avidin-biotinylated liposomes under the same conditions (step D in Fig. 6), that enhanced the electron transfer resistance, respectively, to 17 kΩ and 20 kΩ (curves e and f, in Fig. 7).

In a control experiment, the sensing interface was interacted with mutant, non-complementary DNA **104'** (SEQ ID NO:4, 5x10⁻⁶M), pre-treated with biotinylated oligonucleotide **112** and subsequently treated with avidin and the biotinylated liposome, under the same conditions. A minute increase in the electron-transfer resistance corresponding to Rₑₜ = 3.4 kΩ. was observed, attributed to non-specific adsorption of avidin to the sensing interface.

The increase in the electron-transfer resistance at the electrode upon binding of avidin and the biotin-labeled liposome, were controlled by the bulk concentration of the target-DNA in the sample (Fig. 7B).

Using a double-step avidin/biotin-labeled-liposome amplification pathway, target DNA concentration as low as 5x10⁻¹⁴ (signal to noise ratio S/N=3) was detected.

In a similar manner to that described with reference to Fig. 4A, an oligonucleotide capturing agent was assembled on an Au/quarts crystal. The functionalized interface was then hybridized with a target DNA (concentration 5x10⁻⁶M) followed by interaction thereof with the oligonucleotide-labeled liposome. Fig. 8A (solid line) shows QCM-transduction of the amplified sensing of the analyte. Interaction of the functionalized crystal with the analyte (point a) resulted in a frequency decrease of Δf = 17Hz, implying a surface coverage of the analyte corresponding to 1.2x10⁻¹¹ mole/cm². Further reaction of the double-stranded surface with the oligonucleotide-tagged liposome (point b) resulted in a substantial decrease in the crystal frequency, Δf=-120 Hz.

Fig. 8A shows also the results of a control experiment in which the sensing interface was interacted with the mutated, non-complementary oligonucleotide (5x10⁻⁶ M, point c) followed by treatment with the tagged liposome (point d). As shown, the crystal frequency was unchanged Δf=±2 Hz upon interaction with the non-complementary DNA. Interaction with the tagged liposome slightly altered the crystal frequency, Δf=-5 Hz. This frequency change may be attributed to minute non-specific binding of the liposome to the interface. Association of the amplified oligonucleotide-tagged liposome with the interface resulted in a frequency change of Δf=-70 Hz (point e), that allows the easy amplified detection of the target DNA also by using microgravimetric QCM assay. The lowest sensitivity limit for the detection of the target DNA by this amplification method was estimated to be 5x10-12 M (Δf= -20 Hz, after treatment with tagged liposome).

Fig. 8B shows the results of sensing a target DNA in a sample the manner described in connection with Fig. 6, however also in this case, wherein the a capturing oligonucleotide is assembled on a Au/quarts crystal. Accordingly, first an analyte-double-stranded biotinylated system was associated wit the sensing interface which resulted in a frequency decrease of 25 Hz (curve e). Binding of avidin to the biotinylated assembly yielded a frequency change of Δf∼-50 Hz (point f). Linkage of the biotin-tagged liposomes to the system amplified the primary association of the analyte and a very high frequency change Δf∼-500 Hz was observed (point g). Additional treatment of the interface with avidin, Δf∼-50 Hz (point h) and then with the biotin-labeled liposome (point i) resulted in a second amplification corresponding to Δf=690 Hz.

Treatment of the sensing interface with the biotin-labeled non-complementary DNA did not yield any significant frequency change (point j) and subsequent interaction of the resulting assembly with avidin and the biotin-tagged liposome resulted in a frequency change of about -30 Hz (points k and 1, respectively). As described above, this change in frequency may be attributed to non-specific association of the liposome to the interface. Using the two-step amplification pathway, the lowest sensitivity limit for sensing the target DNA was estimated to be 1x10⁻¹³M (or 1x10⁻¹⁶ mole/ml), which may be further enhanced by performing additional binding steps of avidin-biotinylated liposome.

### SEQUENCE LISTING

<110> YISSUM RESEARCH DEVELOPMENT COMPANY
<120> METHOD AND SYSTEM FOR DETECTING OLIGONUCLEOTIDES IN A SAMPLE
<130> Yissum(Wilner)-1206184
<140> PCT/IL 99/00649
   <141> 1999-12-01
<150> IL127346
   <151> 1998-12-01
<150> IL132966
   <151> 1999-11-16
<160> 9
<170> PatentIn Ver. 2.1
<210> 1
   <211> 13
   <212> DNA
   <213> HUMAN
<400> 1
   tctatcctac gct 13
<210> 2
   <211> 26
   <212> DNA
   <213> HUMAN
<400> 2
   gcgtaggata gatatacggt tcgcgc 26
<210> 3
   <211> 14
   <212> DNA
   <213> HUMAN
<400> 3
   gcgcgaaccg tata 14
<210> 4
   <211> 26
   <212> DNA
   <213> HUMAN
<400> 4
   gcggccatag gatatacggt tcgcgc 26
<210> 5
   <211> 13
   <212> DNA
   <213> HUMAN
<400> 5
   tctatcctac gct 13
<210> 6
   <211> 27
   <212> DNA
   <213> HUMAN
<400> 6
   agcgtaggat agatatacgg ttcgcgc 27
<210> 7
   <211> 14
   <212> DNA
   <213> HUMAN
<400> 7
   gcgcgaaccg tata 14
<210> 8
   <211> 27
   <212> DNA
   <213> HUMAN
<400> 8
   agcgctccag tgatatacgg ttcgcgc 27
<210> 9
   <211> 14
   <212> DNA
   <213> HUMAN
<400> 9

¹ Anmerkung des Übersetzers: im englischen Original steht dafür: "sensing interface"

## Claims

1. A method for detecting a target oligonucleotide in a sample, comprising:
(a) providing a sensor device having a sensing interface carrying capturing oligonucleotides having each a nucleotide sequence complementary in at least a stably hybridizing portion thereof to a first portion of the target oligonucleotides wherein said sensor device comprises an electrochemical probe carrying the sensing interface;
(b) providing verification oligonucleotides having each a nucleotide sequence complementary in at least a stably hybridizing portion thereof to a second portion of the target oligonucleotide, other than said first portion;
(c) contacting the sample with the sensing interface under conditions such as to allow the target oligonucleotides, if present in the sample, to hybridize to the capturing oligonucleotides;
(d) prior to (c) or thereafter, allowing the verification oligonucleotides to hybridize to the target oligonucleotides if present in the sample; and
(e) detecting the presence of said verification oligonucleotides on the sensing interface.

2. The method of Claim 1, wherein said detection is based on Faradaic impedance spectroscopy or amperometric measurements.

3. The method of either of Claims 1 or 2, wherein the sequence complementary to at least a stably hybridizing portion of the target oligonucleotide is of about 12 nucleotides.

4. The method according of any one of Claims 1-3, wherein the verification oligonucleotide is conjugated to a recognition agent which can specifically bind to a signal-amplifying agent, and step (e) of the method comprises:
(e1) contacting the sensing interface with said signal-amplifying agent;
(e2) detecting the presence of said signal-amplifying agent on the sensing interface.

5. The method of Claim 4, wherein said recognition agent is biotin and said signal amplifying agent comprises avidin.

6. The method of any one of Claims 1-3, wherein said verification oligonucleotide is bound to or complexed with a signal-amplifying agent, and step
(e) comprises detecting of presence of the signal-amplifying agent on the sensing interface.

7. The method of any one of Claims 1-3, wherein the verification oligonucleotide comprises a first recognition agent which specifically binds to a recognition partner to form a recognition couple, step (e) of the method comprises the following steps:
(e1) contacting said sensing interface with said recognition partner;
(e2) contacting said sensing interface with a signal-amplifying agent comprising a second recognition agent, which may be the same or different as the first recognition agent, which can also bind to said recognition partner; and
(e3) detecting presence of said signal-amplifying agent on said sensing interface.

8. The method of Claim 7, comprising the following step between steps (e2) and (e3):
(e2.1) repeating steps (e1) and (e2) one or more times.

9. The method of any one of Claims 4-8, wherein said signal-amplifying agent comprises an enzyme which catalyzes a reaction yielding an insoluble reaction product, and step (e) comprises:
(ea) providing conditions permitting catalytic activity of said enzyme to yield formation of said insoluble reaction product; and
(eb) detecting the presence of said insoluble reaction product on said sensing interface.

10. The method of any one of Claims 4-8, wherein said signal-amplifying agent comprises a moiety or a particle which directly increases the mass immobilized on the sensing surface, the method comprises in step (e):
(ea) detecting the presence of said moiety or particle on said sensing interface.

11. The method of Claim 10, wherein said moiety is a molecule or a super molecular structure.

12. A system for detecting a target oligonucleotide in a sample, comprising:
(i) a sensor device having a sensing interface carrying capturing oligonucleotides having each a nucleotide sequence complementary in at least a stably hybridizing portion thereof to a first portion of the target oligonucleotides wherein said sensor device is an electrochemical electrode carrying said sensing interface;
(ii) verification oligonucleotides having each a nucleotide sequence complementary in at least a stably hybridizing portion thereof to a second portion of the target oligonucleotide, other than said first portion; and
(iii) a detecting means comprising one or both of apparatus and reagents for detecting a verification oligonucleotide bound to the sensing interface.

13. The system of Claim 12, wherein when the system comprises an apparatus, said apparatus is adapted for the performance of an electrochemical measurement.

14. The system of either of Claims 12 or 13, wherein said capturing oligonucleotide has a nucleotide sequence complementary to said first portion which has a length of about 12 nucleotides.

15. The system of any one of Claims 12-14, wherein the verification oligonucleotide is conjugated to a recognition agent which specifically binds to a signal-amplifying agent.

16. The system of Claim 15, wherein said recognition agent is biotin and said signal-amplifying agent comprises avidin.

17. The system of any one of Claims 12-14, wherein the verification oligonucleotide is conjugated or complexed with a signal-amplifying agent.

18. A system of any one of Claims 12-14, wherein the verification oligonucleotide is conjugated to a first recognition agent, which specifically binds to a recognition partner, the recognition partner being capable of binding also to a second recognition agent, being the same or different from said first recognition agent; the system further comprises a signal amplifying agent comprising a second recognition agent.

19. A system of Claim 18, when said first and said second recognition agents are biotin and where said recognition partner is avidin or streptavidin.

20. A system of any one of Claims 12-19, wherein said signal-amplifying agent comprises an enzyme which catalyzes a reaction yielding an insoluble reaction product.

21. A system of any one of Claims 12-19, wherein said signal-amplifying agent comprises a particle or moiety which directly increases the mass immobilized on the sensing interface.

22. A method for detecting a target oligonucleotide in a sample, comprising:
(a) providing a sensor device having a sensing interface carrying capturing oligonucleotides having each a nucleotide sequence complementary in at least a stably hybridizing portion thereof to a first portion of the target oligonucleotides;
(b) providing verification oligonucleotides having each a nucleotide sequence complementary in at least a stably hybridizing portion thereof to a second portion of the target oligonucleotide, other than said first portion, wherein the verification oligonucleotide is capable of binding to a signal-amplifying agent comprising a liposome,
(c) contacting the sample with the sensing interface under conditions so as to allow the target oligonucleotides, if present in the sample, to hybridize to the capturing oligonucleotides;
(d) prior to (c) or thereafter, allowing the verification oligonucleotides to hybridize to the target oligonucleotides if present in the sample;
(e) contacting the sensing interface with said signal-amplifying agent; and
(f) detecting the presence of said signal-amplifying agent on the sensing interface.

23. The method of Claim 22 , wherein said sensor device comprises an electrochemical probe carrying the sensing interface.

24. The method of Claim 23, wherein said detection is based on Faradaic impedance spectroscopy or amperometric measurements.

25. The method of Claim 22, wherein said sensor device comprises a microbalance quaitz-crystal probe carrying the sensing interface.

26. The method of Claim 25, wherein said detection is based on a microgravimetric quartz-crystal microbalance (QCM) analysis.

27. The method of any one of Claims 22-26, wherein the sequence complementary to at least a stably hybridizing portion of the target oligonucleotide is of about 12 nucleotides.

28. The method according of any one of Claims 22-27, wherein the verification oligonucleotide is conjugated to a recognition agent which can specifically bind to said signal-amplifying agent.

29. The method of Claim 28 wherein said recognition agent is biotin and said signal amplifying agent comprises avidin.

30. The method of any one of Claims 22-27, wherein said verification oligonucleotide is bound to or complexed with said signal-amplifying agent.

31. The method of any one of Claims 22-27, wherein the verification oligonucleotide comprises a first recognition agent which specifically binds to a recognition partner to form a recognition couple, step (e) of the method comprising the following steps:
(e1) contacting said sensing interface with said recognition partner;
(e2) contacting said sensing interface with said signal-amplifying agent comprising a second recognition agent, which may be the same or different as the frst recognition agent, which can also bind to said recognition partner.

32. The method of Claim 31, comprising the following step after step (e2):
(e2.1) repeating steps (e1) and (e2) one or more times.

33. A system for detecting a target oligonucleotide in a sample, comprising:
(i) a sensor device having a sensing interface carrying capturing oligonucleotides having each a nucleotide sequence complementary in at least a stably hybridizing portion thereof to a first portion of the target oligonucleotides;
(ii) verification oligonucleotides having each a nucleotide sequence complementary in at least a stably hybridizing portion thereof to a second portion of the target oligonucleotide, other than said first portion, wherein the verification oligonucleotide is capable of binding to a signal-amplifying agent comprising a liposome; and
(iii) a detecting means comprising one or both of apparatus and reagent for detecting a verification oligonucleotide bound to the sensing interface, wherein said detecting means comprises said signal-amplifying agent comprising a liposome.

34. The systems of Claim 33, wherein said sensor device is an electrochemical electrode carrying said sensing surface.

35. The system of Claim 33 or 34 wherein said apparatus is adapted for the performance of an electrochemical measurement.

36. The system of Claim 33, wherein said sensor device comprises a microbalance quartz-crystal probe carrying the sensing interface.

37. The system according to Claim 36, wherein said detection is based on a microgravimetric quartz-crystal microbalance (QCM) analysis.

38. The system of Claims 33-37, wherein said capturing oligonucleotide has a nucleotide sequence complementary to said first portion which has a length of about 12 nucleotides.

39. The system of any one of Claims 33-38, wherein the verification oligonucleotide is conjugated to a recognition agent which specifically binds to the signal-amplifying agent.

40. The system of Claim 39, wherein said recognition agent is biotin and said signal-amplifying agent comprises avidin.

41. The system of any one of Claims 33-38, wherein the verification oligonucleotide is conjugated or complexed with the signal-amplifying agent.

42. A system of any one of Claims 33-38, wherein the verification oligonucleotide is conjugated to a first recognition agent, which specifically binds to a recognition partner, the recognition partner being capable of binding also to a second recognition agent, being the same or different from said first recognition agent; the system further comprises the signal amplifying agent comprising a second recognition agent.

43. A system of Claim 42, when said first and said second recognition agents are biotin and where said recognition partner is avidin or streptavidin.

## Patentansprüche

1. Ein Verfahren zum Detektieren eines Target-Oligonukleotids in einer Probe, umfassend:
(a) Bereitstellen einer Sensorvorrichtung, welche eine Grenzfläche mit Sensorfunktion¹ aufweist, die einfangende Oligonukleotide trägt, welche jeweils eine Nukleotidsequenz komplementär in zumindest einer stabil hybridisierenden Portion davon bezogen auf eine erste Portion der Target-Oligonukleotide aufweisen, wobei besagte Sensorvorrichtung eine elektrochemische Sonde umfasst, welche die Grenzfläche mit Sensorfunktion trägt;
(b) Bereitstellen von Verifizierungs-Oligonukleotiden, welche jeweils eine Nukleotidsequenz aufweisen, die komplementär in zumindest einer stabil hybridisierenden Portion davon zu einer zweiten Portion des Target-Oligonukleotids ist, welche von der ersten Portion verschieden ist;
(c) Inkontaktbringen der Probe mit der Grenzfläche mit Sensorfunktion unter Bedingungen, welche der Gestalt sind, dass sie erlauben, dass die Target-Oligonukleotide, falls sie in der Probe vorhanden sind, an die einfangenden Nukleotide hybridisieren;
(d) Ermöglichen, dass die Verifikations-Oligonukleotide vor dem Schritt (c) oder danach an die Target-Oligonukleotide, wenn sie in der Probe vorhanden sind, hybridisieren; und
(e) Detektieren der Gegenwart von besagten Verifikations-Oligonukleotiden auf der Grenzfläche mit Sensorfunktion.

2. Das Verfahren von Anspruch 1, worin besagte Detektion auf Faradaischer Impedanz-Spektroskopie oder auf amperometrischen Messungen basiert.

3. Das Verfahren von einem der Ansprüche 1 oder 2, worin die Sequenz, welche komplementär zu zumindest einer stabil hybridisierenden Portion des Target-Oligonukleotids ist, etwa 12 Nukleotiden aufweist.

4. Das Verfahren gemäß irgendeinem der Ansprüche 1 bis 3, worin das Vertifikations-Oligonukleotid an ein Erkennungsagens konjugiert ist, welches spezifisch an ein Signal amplifizierendes Agens binden kann, und Schritt (e) des Verfahrens folgendes umfasst:
(e1) Inkontaktbringen der Grenzfläche mit Sensorfunktion mit besagtem Signal-amplifizierendem Agens;
(e2) Detektieren des Vorhandenseins von besagtem Signal-amplifizierendem Agens auf der Grenzfläche mit Sensorfunktion.

5. Das Verfahren nach Anspruch 4, worin besagtes Erkennungsagens Biotin ist und besagtes Signal-amplifizierendes Agens Avidin umfasst.

6. Das Verfahren von irgendeinem der Ansprüche 1 bis 3, worin besagtes Vertifikations-Oligonukleotid gebunden an oder komplexiert mit einem Signal-amplifizierenden Agens ist und Schritt (e) das Detektieren der Gegenwart des Signal-amplifizierenden Agens auf der Grenzfläche mit Sensorfunktion umfasst.

7. Das Verfahren von irgendeinem der Ansprüche 1 bis 3, worin das Verifikations-Oligonukleotid ein erstes Erkennungsagens umfasst, welches spezifisch an einen Erkennungspartner bindet, um ein Erkennungspaar zu bilden, und Schritt (e) des Verfahrens die folgenden Schritte umfasst:
(e1) Inkontaktbringen besagter Grenzfläche mit Sensorfunktion mit besagtem Erkennungspartner;
(e2) Inkontaktbringen besagter Grenzfläche mit Sensorfunktion mit einem Signal-amplifizierenden Agens, welches ein zweites Erkennungsagens umfasst, welches das gleiche oder verschieden von dem ersten Erkennungsagens sein kann, welches auch an besagten Erkennungspartner binden kann; und
(e3) Detektieren der Gegenwart von besagtem Signal-amplifizierenden Agens auf besagter Grenzfläche mit Sensorfunktion.

8. Das Verfahren von Anspruch 7, umfassend den folgenden Schritt zwischen den Schritten (e2) und (e3):
(e2.1) Wiederholen der Schritte (e1) und (e2) ein oder mehrere Male.

9. Das Verfahren von irgendeinem der Ansprüche 4 bis 8, worin besagtes Signal-amplifizierendes Agens ein Enzym umfasst, welches eine Reaktion katalysiert, die ein unlösliches Reaktionsprodukt hervorbringt und Schritt (e) umfasst:
(ea) Bereitstellen von Bedingungen, welche katalytische Aktivität von besagtem Enzym ermöglichen, zur Bildung von besagtem unlöslichem Reaktionsprodukt zu führen; und
(eb) Detektieren der Gegenwart von besagtem unlöslichem Reaktionsprodukt auf besagter Grenzfläche mit Sensorfunktion.

10. Das Verfahren von irgendeinem der Ansprüche 4 bis 8, worin besagtes Signal-amplifizierendes Agens eine Gruppe oder ein Partikel umfasst, welches direkt die Masse, welche auf der Grenzfläche mit Sensorfunktion immobilisiert ist, vergrößert, und das Verfahren in Schritt (e) umfasst:
(ea) Detektieren der Gegenwart von besagter Gruppe oder besagtem Partikel auf besagter Grenzfläche mit Sensorfunktion.

11. Das Verfahren von Anspruch 10, worin besagte Gruppe ein Molekül ist oder eine supermolekulare Struktur.

12. Ein System zum Detektieren eines Target-Oligonukleotids in einer Probe, umfassend:
(i) eine Sensorvorrichtung, welche eine Grenzfläche mit Sensorfunktion aufweist, die einfangende Oligonukleotide trägt, wobei ein jedes eine Nukleotidsequenz komplementär in zumindest einer stabilen hybridisierenden Portion davon, bezogen auf eine erste Portion des Target-Oligonukleotids aufweist, wobei besagte Sensorvorrichtung eine elektrochemische Elektrode ist, welche besagte Grenzfläche mit Sensorfunktion trägt;
(ii) Verifikations-Oligonukleotide, welche jeweils eine Nukleotidsequenz aufweisen, die komplementär in zumindest einer stabil hybridisierenden Portion davon, bezogen auf eine zweite Portion des Target-Oligonukleotids ist, welche verschieden von besagter erster Portion ist; und
(iii) ein Detektionsmittel, welches einen oder beide, Apparate und Reagenzien zur Detektion eines Verifikations-Oligonukleotids, welches an die Grenzfläche mit Sensorfunktion gebunden ist, umfasst.

13. Das System von Anspruch 12, worin wenn das System einen Apparat umfasst, besagter Apparat adaptiert ist für die Durchführung einer elektrochemischen Messung.

14. Das System entweder von Anspruch 12 oder 13, worin besagtes einfangendes Olgonukleotid eine Nukleotidsequenz aufweist, welche komplementär zu besagter erster Portion ist, welche eine Länge von ungefähr 12 Nukleotiden aufweist.

15. Das System von irgendeinem der Ansprüche 12 bis 14, worin das Verifikations-Oligonukleotid konjugiert zu einem Erkennungsagens ist, welches spezifisch an ein Signal-amplifizierendes Agens bindet.

16. Das System von Anspruch 15, worin besagtes Erkennungsagens Biotin ist und besagtes amplifizierendes Agens Avidin umfasst.

17. Das System von irgendeinem der Ansprüche 12 bis 14, worin das Verifikations-Oligonukleotid konjugiert oder komplexiert mit einem Signal-amplifizierenden Agens ist.

18. Ein System von irgendeinem der Ansprüche 12 bis 14, worin das Verifikations-Oligonukleotid konjugiert an ein erstes Erkennungsagens ist, welches spezifisch an einen Erkennungspartner bindet, wobei der Erkennungspartner in der Lage ist, auch an ein zweites Erkennungsagens zu binden, welcher der gleiche oder verschieden von dem ersten Erkennungsagens ist; wobei das System des weiteren ein Signal-amplifizierendes Agens umfasst, welches ein zweites Erkennungsagens umfasst.

19. Ein System von Anspruch 18, worin besagte erste und besagte zweite Erkennungsagentien Biotin sind und worin besagter Erkennungspartner Avidin oder Streptavidin ist.

20. Ein System von irgendeinem der Ansprüche 12 bis 19, worin besagtes Signal-amplifizierendes Agens ein Enzym umfasst, welches eine Reaktion katalysiert, die ein unlösliches Reaktionsprodukt erzeugt.

21. Ein System von irgendeinem der Ansprüche 12 bis 19, worin besagtes Signal-amplifizierendes Agens ein Partikel oder eine Gruppe umfasst, welche(s) direkt die immobilisierte Masse auf der Grenzfläche mit Sensorfunktion vergrößert.

22. Ein Verfahren zum Detektieren eines Target-Oligonukleotids in einer Probe, umfassend:
(a) Bereitstellen einer Sensorvorrichtung, welche eine Grenzfläche mit Sensorfunktion aufweist, die einfangende Oligonukleotide trägt, welche jeweils eine Nukleotidsequenz komplementär in zumindest einer stabil hybridisierenden Portion davon bezogen auf eine erste Portion der Target-Oligonukleotide aufweisen;
(b) Bereitstellen von Verifizierungs-Oligonukleotiden, welche jeweils eine Nukleotidsequenz aufweisen, die komplementär in zumindest einer stabil hybridisierenden Portion davon zu einer zweiten Portion des Target-Oligonukleotids ist, welche von der ersten Portion verschieden ist, worin das Verifikations-Oligonukleotid in der Lage ist, an ein Signal-amplifizierendes Agens zu binden, welches ein Liposom umfasst,
(c) Inkontaktbringen der Probe mit der Grenzfläche mit Sensorfunktion unter Bedingungen, welche der Gestalt sind, dass sie erlauben, dass die Target-Oligonukleotide, falls sie in der Probe vorhanden sind, an die einfangenden Nukleotide hybridisieren;
(d) Ermöglichen, dass die Verifikations-Oligonukleotide vor dem Schritt (c) oder danach an die Target-Oligonukleotide, wenn sie in der Probe vorhanden sind, hybridisieren;
(e) Kontaktieren der Grenzfläche mit Sensorfunktion mit besagtem Signal-amplifizierendem Agens; und
(f) Detektieren der Gegenwart von besagten Verifikations-Oligonukleotiden auf der Grenzfläche mit Sensorfunktion.

23. Das Verfahren von Anspruch 22, worin besagte Sensorvorrichtung eine elektrochemische Sonde umfasst, welche die Grenzfläche mit Sensorfunktion trägt.

24. Das Verfahren von Anspruch 23, worin besagte Detektion auf Faradaischer Impedanz-Spektroskopie oder auf amperometrischen Messungen basiert.

25. Das Verfahren von Anspruch 22, worin besagte Sensorvorrichtung eine Mikrobalance-Quarz-Kristallsonde umfasst, welche die Grenzfläche mit Sensorfunktion trägt.

26. Das Verfahren von Anspruch 25, worin besagte Detektion auf mikrogravimetrischer Quarz-Kristall-Mikrobalance-Analyse (QCM), (quarz-crystal micorbalance) basiert.

27. Das Verfahren von irgendeinem der Ansprüche 22 bis 26, worin die Sequenz komplementär zu zumindest einer stabil hybridisierenden Portion des Target-Oligonukleoüds etwa 12 Nukleotiden aufweist.

28. Das Verfahren gemäß irgendeinem der Ansprüche 22 bis 27, worin das Verifikations-Oligonukleotid an ein Erkennungsagens konjugiert ist, welches spezifisch an besagtes Signal-amplifizierendes Agens binden kann.

29. Das Verfahren von Anspruch 28, worin besagtes Erkennungsagens Biotin ist und besagtes Signal-amplifizierendes Agens Avidin umfasst.

30. Das Verfahren von irgendeinem der Ansprüche 22 bis 27, worin besagtes Verifikations-Oligonukleotid gebunden oder komplexiert ist mit besagtem Signal-amplifizierendem Agens.

31. Das Verfahren von irgendeinem der Ansprüche 22 bis 27, worin das Venfikations-Oligonukleotid ein erstes Erkennungsagens umfasst, welches spezifisch an einen Erkennungspartner bindet, um ein Erkennungspaar zu bilden, und Schritt (e) des Verfahrens die folgenden Schritte umfasst:
(e1) Inkontaktbringen besagter Grenzfläche mit Sensorfunktion mit besagtem Erkennungspartner;
(e2) Inkontaktbringen besagter Grenzfläche mit Sensorfunktion mit einem Signal-amplifizierenden Agens, welches ein zweites Erkennungsagens umfasst, welches das gleiche oder verschieden von dem ersten Erkennungsagens sein kann, welches auch an besagten Erkennungspartner binden kann.

32. Das Verfahren von Anspruch 31, umfassend den folgenden Schritt nach dem Schritt (e2):
(e2.1) wiederholen der Schritte (e1) und (e2) ein oder mehrere Male.

33. Ein System zur Detektion eines Target-Oligonukleotids in einer Probe, umfassend:
(i) eine Sensorvorrichtung, welche eine Grenzfläche mit Sensorfunktion aufweist, welche einfangende Oligonukleotide trägt, welche jeweils eine Nukleotidsequenz komplementär in zumindest einer stabilen hybridisierenden Portion davon bezogen auf eine erste Portion des Target-Oligonukleotids aufweisen;
(ii) Veritikations-Oligonukleotide, welche jeweils eine Nukleotidsequenz aufweisen, die komplementär in zumindest einer stabil hybridisierenden Portion davon, bezogen auf eine zweite Portion des Target-Oligonukleotids, welche verschieden von besagter erster Portion ist, aufweisen, worin das Verifikations-Oligonukleotid in der Lage ist, an ein Signal-amplifizierendes Agens zu binden, welches ein Liposom umfasst; und
(iii) ein Detektionsmittel, welches einen oder beide, Apparate und Reagenzien zur Detektion eines Verifikationsoligonukleotids, welches an die Grenzfläche mit Sensorfunktion gebunden ist, umfasst, worin besagtes Detektionsmittel ein Signal-amplifizierendes Agens umfasst, welches ein Liposom umfasst.

34. Das System von Anspruch 33, worin besagte Sensorvorrichtung eine elektrochemische Elektrode ist, welche besagte Grenzfläche mit Sensorfunktion trägt.

35. Das System von Anspruch 33 oder 34, worin besagter Apparat adaptiert ist für die Durchführung einer elektrochemischen Messung.

36. Das System von Anspruch 33, worin besagte Sensorvorrichtung eine Microbalance-Quarz-Kristallsonde umfasst, welche die Grenzfläche mit Sensorfunktion trägt.

37. Das System gemäß Anspruch 36, worin besagte Detektion auf mikrogravimetrischer Quarz-Kristall-Mikrobalance-Analyse (QCM, quarz-crystal micorbalance) basiert.

38. Das System von Ansprüchen 33 bis 37, worin besagtes einfangendes Oligonukleotid eine Nukleotidsequenz aufweist, welche komplementär zu besagter erster Portion ist, welche eine Länge von ungefähr 12 Nukleotiden aufweist.

39. Das System von irgendeinem der Ansprüche 33 bis 38, worin das Verifikations-Oligonukleotid konjugiert an ein Erkennungsagens ist, welches spezifisch an das Signal-amplifizierende Agens bindet.

40. Das System von Anspruch 39, worin besagtes Erkennungsagens Biotin ist und besagtes Signal-amplifizierendes Agens Avidin umfasst.

41. Das System von irgendeinem der Ansprüche 33 bis 38, worin das Verifikations-Oligonukleotid konjugiert oder komplexiert mit dem Signal-amplifizierenden Agens ist.

42. Ein System von irgendeinem der Ansprüche 33 bis 38, worin das Verifikations-Oligonukleotid konjugiert an ein erstes Erkennungsagens ist, welches spezifisch an einen Erkennungspartner bindet, wobei der Erkennungspartner in der Lage ist, auch an ein zweites Erkennungsagens zu binden, welcher der gleiche oder verschieden von dem ersten Erkennungsagens ist; wobei das System des weiteren ein Signal-amplifizierendes Agens umfasst, welches ein zweites Erkennungsagens umfasst.

43. Ein System von Anspruch 42, worin besagte erste und besagte zweite Erkennungsagenzien Biotin sind und worin besagter Erkennungspartner Avidin oder Streptavidin ist.

## Revendications

1. Procédé pour détecter un oligonucléotide cible dans un échantillon, comprenant :
(a) la fourniture d'un détecteur ayant une interface sensible portant des oligonucléotides de capture ayant chacun une séquence de nucléotides complémentaire, dans au moins une portion d'hybridation stable de ceux-ci, d'une première portion des oligonucléotides cibles, dans lequel ledit détecteur comprend une sonde électrochimique portant l'interface sensible ;
(b) la fourniture d'oligonucléotides de vérification ayant chacun une séquence de nucléotides complémentaire, dans au moins une portion d'hybridation stable de ceux-ci, d'une deuxième portion de l'oligonucléotide cible, autre que ladite première portion ;
(c) la mise en contact de l'échantillon avec l'interface sensible dans des conditions telles qu'elles permettent aux oligonucléotides cibles, s'ils sont présents dans l'échantillon, de s'hybrider aux oligonucléotides de capture ;
(d) préalablement à (c) ou par la suite, la possibilité pour les oligonucléotides de vérification de s'hybrider aux oligonucléotides cibles s'ils sont présents dans l'échantillon ; et
(e) la détection de la présence desdits oligonucléotides de vérification sur l'interface sensible.

2. Procédé selon la revendication 1, dans lequel ladite détection est basée sur la spectroscopie d'impédance faradique ou des mesures ampérométriques.

3. Procédé selon l'une ou l'autre des revendications 1 et 2, dans lequel la séquence complémentaire d'au moins une portion d'hybridation stable de l'oligonucléotide cible est d'environ 12 nucléotides.

4. Procédé selon l'une quelconque des revendications 1 à 3, dans lequel l'oligonucléotide de vérification est conjugué à un agent de reconnaissance qui peut se lier spécifiquement à un agent d'amplification de signal, et l'étape (e) du procédé comprend :
(e1) la mise en contact de l'interface sensible avec ledit agent d'amplification de signal ;
(e2) la détection de la présence dudit agent d'amplification de signal sur l'interface sensible.

5. Procédé selon la revendication 4, dans lequel ledit agent de reconnaissance est la biotine et ledit agent d'amplification de signal comprend l'avidine.

6. Procédé selon l'une quelconque des revendications 1 à 3, dans lequel ledit oligonucléotide de vérification est lié à ou complexé avec un agent d'amplification de signal, et l'étape (e) comprend la détection de la présence de l'agent d'amplification de signal sur l'interface sensible.

7. Procédé selon l'une quelconque des revendications 1 à 3, dans lequel l'oligonucléotide de vérification comprend un premier agent de reconnaissance qui se lie spécifiquement à un partenaire de reconnaissance pour former un couple de reconnaissance, et l'étape (e) du procédé comprend les étapes suivantes :
(e1) la mise en contact de l'interface sensible avec ledit partenaire de reconnaissance ;
(e2) la mise en contact de ladite interface sensible avec un agent d'amplification de signal comprenant un deuxième agent de reconnaissance, qui peut être le même ou différent du premier agent de reconnaissance, qui peut aussi se lier audit partenaire de reconnaissance ; et
(e3) la détection de la présence dudit agent d'amplification de signal sur ladite interface sensible.

8. Procédé selon la revendication 7, comprenant l'étape suivante entre les étapes (e2) et (e3) :
(e2.1) la répétition des étapes (e1) et (e2) une ou plusieurs fois.

9. Procédé selon l'une quelconque des revendications 4 à 8, dans lequel ledit agent d'amplification de signal comprend une enzyme qui catalyse une réaction donnant un produit de réaction insoluble, et l'étape (e) comprend :
(ea) la fourniture de conditions autorisant l'activité catalytique de ladite enzyme pour donner la formation dudit produit de réaction insoluble ; et
(eb) la détection de la présence dudit produit de réaction insoluble sur ladite interface sensible.

10. Procédé selon l'une quelconque des revendications 4 à 8, dans lequel ledit agent d'amplification de signal comprend un motif ou une particule qui augmente directement la masse immobilisée sur la surface sensible, le procédé comprenant dans l'étape (e) :
(ea) la détection de la présence dudit motif ou particule sur ladite interface sensible.

11. Procédé selon la revendication 10, dans lequel ledit motif est une molécule ou une structure supermoléculaire.

12. Système pour détecter un oligonucléotide cible dans un échantillon, comprenant :
(i) un détecteur ayant une interface sensible portant des oligonucléotides de capture ayant chacun une séquence de nucléotides complémentaire, dans au moins une portion d'hybridation stable de ceux-ci, d'une première portion des oligonucléotides cibles, dans lequel ledit détecteur est une électrode électrochimique portant ladite interface sensible ;
(ii) des oligonucléotides de vérification ayant chacun une séquence de nucléotides complémentaire, dans au moins une portion d'hybridation stable de ceux-ci, d'une deuxième portion de l'oligonucléotide cible, autre que ladite première portion ; et
(iii) des moyens de détection comprenant l'un ou l'autre, ou les deux, d'un appareil et de réactifs pour détecter un oligonucléotide de vérification lié à l'interface sensible.

13. Système selon la revendication 12, dans lequel le système comprend un appareil, ledit appareil étant adapté pour effectuer une mesure électrochimique.

14. Système selon l'une ou l'autre des revendications 12 et 13, dans lequel ledit oligonucléotide de capture a une séquence de nucléotides complémentaire de ladite première portion qui a une longueur d'environ 12 nucléotides.

15. Système selon l'une quelconque des revendications 12 à 14, dans lequel l'oligonucléotide de vérification est conjugué à un agent de reconnaissance qui se lie spécifiquement à un agent d'amplification de signal.

16. Système selon la revendication 15, dans lequel ledit agent de reconnaissance est la biotine et ledit agent d'amplification de signal comprend l'avidine.

17. Système selon l'une quelconque des revendications 12 à 14, dans lequel l'oligonucléotide de vérification est conjugué à ou complexé avec un agent d'amplification de signal.

18. Système selon l'une quelconque des revendications 12 à 14, dans lequel l'oligonucléotide de vérification est conjugué à un premier agent de reconnaissance, qui se lie spécifiquement à un partenaire de reconnaissance, le partenaire de reconnaissance étant capable de se lier aussi à un deuxième agent de reconnaissance, qui est le même ou différent dudit premier agent de reconnaissance ; le système comprenant en outre un agent d'amplification de signal comprenant un deuxième agent de reconnaissance.

19. Système selon la revendication 18, où lesdits premier et deuxième agents de reconnaissance sont la biotine et où ledit partenaire de reconnaissance est l'avidine ou la streptavidine.

20. Système selon l'une quelconque des revendications 12 à 19, dans lequel ledit agent d'amplification de signal comprend une enzyme qui catalyse une réaction donnant un produit de réaction insoluble.

21. Système selon l'une quelconque des revendications 12 à 19, dans lequel ledit agent d'amplification de signal comprend une particule ou un motif qui augmente directement la masse immobilisée sur l'interface sensible.

22. Procédé pour détecter un oligonucléotide cible dans un échantillon, comprenant :
(a) la fourniture d'un détecteur ayant une interface sensible portant des oligonucléotides de capture ayant chacun une séquence de nucléotides complémentaire, dans au moins une portion d'hybridation stable de ceux-ci, d'une première portion des oligonucléotides cibles ;
(b) la fourniture d'oligonucléotides de vérification ayant chacun une séquence de nucléotides complémentaire, dans au moins une portion d'hybridation stable de ceux-ci, d'une deuxième portion de l'oligonucléotide cible, autre que ladite première portion, dans laquelle l'oligonucléotide de vérification est capable de se lier à un agent d'amplification de signal comprenant un liposome ;
(c) la mise en contact de l'échantillon avec l'interface sensible dans des conditions telles qu'elles permettent aux oligonucléotides cibles, s'ils sont présents dans l'échantillon, de s'hybrider aux oligonucléotides de capture ;
(d) préalablement à (c) ou par la suite, la possibilité pour les oligonucléotides de vérification de s'hybrider aux oligonucléotides cibles s'ils sont présents dans l'échantillon ; et
(e) la mise en contact de l'interface sensible avec l'agent d'amplification de signal ; et
(f) la détection de la présence dudit agent d'amplification de signal sur l'interface sensible.

23. Procédé selon la revendication 22, dans lequel ledit détecteur comprend une sonde électrochimique portant l'interface sensible.

24. Procédé selon la revendication 23, dans lequel ladite détection est basée sur la spectroscopie d'impédance faradique ou des mesures ampérométriques.

25. Procédé selon la revendication 22, dans lequel ledit détecteur comprend une sonde à microbalance à cristal de quartz portant l'interface sensible.

26. Procédé selon la revendication 25, dans lequel ladite détection est basée sur une analyse microgravimétrique à microbalance à cristal de quartz (QCM).

27. Procédé selon l'une quelconque des revendications 22 à 26, dans lequel la séquence complémentaire d'au moins une portion d'hybridation stable de l'oligonucléotide cible est d'environ 12 nucléotides.

28. Procédé selon l'une quelconque des revendications 22 à 27, dans lequel l'oligonucléotide de vérification est conjugué à un agent de reconnaissance qui peut se lier spécifiquement audit agent d'amplification de signal.

29. Procédé selon la revendication 28, dans lequel ledit agent de reconnaissance est la biotine et ledit agent d'amplification de signal comprend l'avidine.

30. Procédé selon l'une quelconque des revendications 22 à 27, dans lequel ledit oligonucléotide de vérification est lié à ou complexé avec ledit agent d'amplification de signal.

31. Procédé selon l'une quelconque des revendications 22 à 27, dans lequel l'oligonucléotide de vérification comprend un premier agent de reconnaissance qui se lie spécifiquement à un partenaire de reconnaissance pour former un couple de reconnaissance, et l'étape (e) du procédé comprend les étapes suivantes :
(e1) la mise en contact de l'interface sensible avec ledit partenaire de reconnaissance ;
(e2) la mise en contact de ladite interface sensible avec ledit agent d'amplification de signal comprenant un deuxième agent de reconnaissance, qui peut être le même ou différent du premier agent de reconnaissance, qui peut aussi se lier audit partenaire de reconnaissance.

32. Procédé selon la revendication 31, comprenant l'étape suivante après l'étape (e2) :
(e2.1) la répétition des étapes (e1) et (e2) une ou plusieurs fois.

33. Système pour détecter un oligonucléotide cible dans un échantillon, comprenant :
(i) un détecteur ayant une interface sensible portant des oligonucléotides de capture ayant chacun une séquence de nucléotides complémentaire, dans au moins une portion d'hybridation stable de ceux-ci, d'une première portion des oligonucléotides cibles ;
(ii) des oligonucléotides de vérification ayant chacun une séquence de nucléotides complémentaire, dans au moins une portion d'hybridation stable de ceux-ci, d'une deuxième portion de l'oligonucléotide cible, autre que ladite première portion, dans lequel l'oligonucléotide de vérification est capable de se lier à un agent d'amplification de signal comprenant un liposome ; et
(iii) des moyens de détection comprenant l'un ou l'autre, ou les deux, d'un appareil et de réactifs pour détecter un oligonucléotide de vérification lié à l'interface sensible, dans lequel lesdits moyens de détection comprennent ledit agent d'amplification de signal comprenant un liposome.

34. Système selon la revendication 33, dans lequel le détecteur est une électrode électrochimique portant ladite surface sensible.

35. Système selon la revendication 33 ou 34, dans lequel ledit appareil est adapté pour effectuer une mesure électrochimique.

36. Système selon la revendication 33, dans lequel ledit détecteur comprend une sonde à microbalance à cristal de quartz portant l'interface sensible.

37. Système selon la revendication 36, dans lequel ladite détection est basée sur une analyse microgravimétrique à microbalance à cristal de quartz (QCM).

38. Système selon les revendications 33 à 37, dans lequel ledit oligonucléotide de capture a une séquence de nucléotides complémentaire de ladite première portion qui a une longueur d'environ 12 nucléotides.

39. Système selon l'une quelconque des revendications 33 à 38, dans lequel l'oligonucléotide de vérification est conjugué à un agent de reconnaissance qui se lie spécifiquement à l'agent d'amplification de signal.

40. Système selon la revendication 39, dans lequel ledit agent de reconnaissance est la biotine et ledit agent d'amplification de signal comprend l'avidine.

41. Procédé selon l'une quelconque des revendications 33 à 38, dans lequel l'oligonucléotide de vérification est conjugué à ou complexé avec l'agent d'amplification de signal.

42. Système selon l'une quelconque des revendications 33 à 38, dans lequel l'oligonucléotide de vérification est conjugué à un premier agent de reconnaissance, qui se lie spécifiquement à un partenaire de reconnaissance, le partenaire de reconnaissance étant capable de se lier aussi à un deuxième agent de reconnaissance, qui est le même ou différent dudit premier agent de reconnaissance ; le système comprenant en outre l'agent d'amplification de signal comprenant un deuxième agent de reconnaissance.

43. Système selon la revendication 42, où lesdits premier et deuxième agents de reconnaissance sont la biotine et où ledit partenaire de reconnaissance est l'avidine ou la streptavidine.
